# EUROPEAN PATENT APPLICATION

(11) **EP 2 050 401 A1**
(43) Date of publication of application: **22.04.2009**
(21) Application number: 07020528.1
(22) Date of filing: 19.10.2007
(51) Int. Cl.: A61B 17/22

(54) **Lithotripter with a holding arm**

(71) Applicant: Dornier MedTech Systems GmbH, 82234 Wessling (DE)
(72) Inventor: Buchbauer, Peter, 85748 Garching (DE)
(74) Representative: Grünecker, Kinkeldey, Stockmair & Schwanhäusser Anwaltssozietät

(57) **Abstract**

The present invention relates to a lithotripter (1) with a base unit (2) and a C-shaped holding arm assembly (3) supporting a therapy head (4) wherein a horizontal joint (5) is provided by means of which the therapy head is pivotable between an over-table position and an under-table position. To improve the movement possibilities of the therapy head while having a construction which is as easy as possible, it is suggested to provide a holding arm assembly comprising arm segments (7,8,10) pivotably joined with respect to each other in a way that the therapy head is movable between a first lateral treatment position facing the base unit and a second lateral treatment position opposing the base unit with respect to a patient.

## Description

The present invention relates to a lithotripter with a base unit and a C-shaped holding arm assembly supporting a therapy head.

A generic lithotripter is known from DE 103 37 523 A1. The lithotripter comprises a holding arm assembly in which a therapy head is pivotable between an over-table and under-table position by means of a horizontal joint provided between a cross arm and a C-shaped holding arm supporting the therapy head. Hereby, the therapy head is movable on a predetermined path in an easy manner.

DE 10 2004 010 004 A1 relates to a lithotripter with a C-arm. A slide is movable along the inside of the C-arm and carries a holding arm supporting a therapy head. Via the slide the therapy head can be moved from a position over a patient table to a position under the patient table on a predetermined path.

From WO 2005/082261 A1 a completely different concept is known in which a lithotripter is provided with a robot arm as a holding arm assembly. The robot arm allows free movement of a therapy head connected hereto. However, a complicated control is necessary in order to move the therapy head on a desired path.

Although the robot arm is not a C-shaped holding arm assembly, there is good access to a circumferential area so that there is enough working space for medical personnel around the therapy head even if same is positioned within a recess of a patient table.

DE 195 12 958 A1 relates to a lithotripter device in which the height of a therapy head with respect to a patient table is adjusted by means of a vertical slide assembly. The therapy head can only be positioned over the patient table as the vertical slide ends approximately at the level of the patient table and the space between the patient table and the vertical slide is too narrow to pass the holding arm assembly and therapy head through. It would be necessary to prolong the vertical joint and to provide a lateral recess in the patient table to be able to lower the therapy head below the patient table in order to have a further possible position in which the therapy head can be used.

It is the object of the present invention to provide an improved generic lithotripter with good movement possibilities of the therapy head while having a construction that is as easy as possible.

According to the present invention, the object is solved by a lithotripter having the features of independent claim 1.

As it is possible to position the therapy head in the two lateral treatment positions, it becomes possible to treat a patient on the side facing the base unit as well as on the side opposing the base unit. Hereby, a bilateral treatment of a patient can be carried out faster and more comfortably with an easy construction of the holding arm assembly.

Advantageously, the holding arm assembly may comprise at least two arm segments that are pivotable in a scissor-like manner. This is an easy manner for prolonging and shortening the holding arm assembly. If the holding arm assembly is prolonged, e.g. with said two arm segments being in a generally stretched relative position, the therapy head can be positioned in the second lateral treatment position. If the holding arm assembly is shortened, e.g. with said two arm segments being in a generally folded-up state, the therapy head can be in the first lateral treatment position.

Favourably, a scissor-like relative movement of at least two arm segments of the holding arm assembly can be carried out along a hypothetical sphere layer surrounding a region in which the patient is to be treated. This means that the scissor-like movement of the arm segments takes place cross to said region in which the patient is to be treated. It is possible to have a construction in which the distance of the arm segments to said region remains basically constant during the scissor-like relative movement.

Advantageously, the axis of at least one joint pivotably connecting two arm segments in a scissor-like manner is generally directed to a region in which the patient is to be treated. The distance between the arm segment pivoting around said axis and said region remains basically constant. Said arm segment is moved generally cross to said region.

Particularly preferable, at least two arm sections that are pivotable in a scissor-like manner are positioned consecutively in a direction towards a region in which a patient is to be treated, at least when these arm segments are in a folded-up relative position. Accordingly, the at least two arm segments can be arranged in a kind of row in a direction towards said region, at least when the arm segments are in,a folded up relative position.

Advantageously, the axis of at least one joint pivotably connecting two arm segments in a scissor-like manner is movable along a cone shell. In this manner, said axis remains basically directed to a certain area during its movement.

Particularly preferred, a pair of two arm segments that are pivotable in a scissor-like manner is pivotably provided between the base unit and an arm segment that supports the therapy head. In such a construction, the first arm segment can remain in its pivotal position when the holding arm assembly is prolonged or shortened. Hereby, the adjustment of the length of the therapy head is easier and more comfortable.

Particularly advantageous, the axes of the joints connecting said three arm segments are generally directed to a region in which the patient is to be treated. Accordingly, the elements pivoted around these axes can basically remain at their distance to said region. Said elements can basically be moved cross to said region. Herewith, the movement around said axes in sum is carried out with the elements basically maintaining their distance to said region and moving basically cross to said region.

Preferably, the axes of the joints connecting said three arm segments and the horizontal axis can be moved into one plane. In this position, the three joints are in a straight row-like arrangement.

Favourably, said axes can be in one plane when the therapy head is in the first lateral treatment position and/or when the therapy head is in the second lateral treatment position. This means that the straight row-like arrangement of the three axes can be achieved in at least one lateral treatment position.

Particularly advantageous, the plane in which said axes can be when the therapy head is in the first lateral treatment position and the plane in which said axis can be when the therapy head is in the second therapy treatment position are identical. Accordingly, it is possible to have the holding assembly in a state with prolonged effective length and in a state with shortened effective with said axes being in the same plane. This can mean practically that the section of the holding arm assembly extending between a first segment and a horizontal joint can have basically the same orientation in the two lateral treatment positions.

Advantageously, the axis of the joint connecting the arm segments constituting said pair of two arm segments can be moved out of said one plane while the other axis remains in said one plane, preferably when the therapy head is in the first lateral treatment position. That is, the position of said joint can be changed, e.g. if the space at its present position is needed, while the other joint connecting arm segments can remain in its position.

Particularly preferable, the joints linking said three arm segments are provided in sections of hypothetical consecutive shells, wherein one shell section accommodates the other shell section at least partially. That is, the three arm segments may be positioned and/or movable in accordance with different shell section-like layers.

Preferably, the horizontal joint and at least one joint linking arm segment are provided in sections of hypothetical consecutive shells, wherein one shell section accommodates the other shell section at least partially. Accordingly, the joints may be positioned and/or movable in accordance with different shell section-like layers.

Advantageously, the holding arm assembly comprises an isocentric kinematic by means of which the therapy head is isocentrically movable between the first and second treatment position. If a desired adjustment of patient and isocentre is achieved, the adjustment of said therapy head to patient is maintained even if the therapy head is moved between a first and second treatment position.

Favourably, the horizontal axis and all axes of joints linking arm segments of the holding arm assembly pass through a focus of the therapy head. Accordingly, the focus coincides with the isocentre.

An embodiment of the invention is shown in the drawing and is described below. In the drawing the following is shown:
- Fig. 1: is a perspective view of an inventive lithotripter with the therapy head being in a first lateral treatment position and being in an under-table position,
- Fig. 2: the lithotripter with the therapy head being in another under-table position,
- Fig. 3: the lithotripter with the therapy head being in a first lateral treatment position and being in an over-table position,
- Fig. 4: the lithotripter with the therapy head being in a second lateral treatment position and being in an over-table position,
- Fig. 5: the lithotripter with the therapy head being in a second lateral treatment position and being in an under-table position, and
- Fig. 6: the lithotripter with the therapy head being in another under-table position.

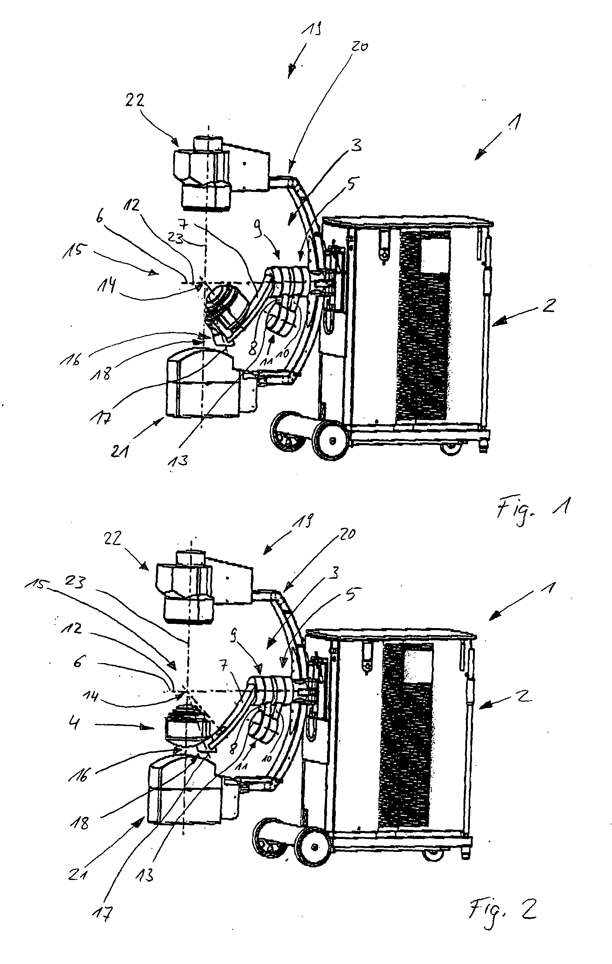

Fig. 1 shows an inventive lithotripter 1 which has a base unit 2 and a C-shape type holding arm assembly 3 supporting a therapy head 4. The holding arm assembly 3 is pivotably connected to the base unit 2 via a horizontal joint 5 so that the holding arm assembly 3 and the therapy head 4 can be pivoted about a horizontal axis 6 between over-table positions and under-table positions. In an over-table position the therapy head 4 is applied to a patient from a position above a patient table and in an under-table position, the therapy head is applied to a patient in a position below the patient table.

The holding arm assembly comprises three arm segments that are pivotably connected to each other. A first arm segment 7 caries the therapy head 4 and is connected to a second arm segment 8 via a first arm segment joint 9. The second arm segment 8 is connected to a third arm segment 10 via a second arm segment joint 11. The third arm segment 10 is connected to the base unit 2 via said horizontal joint 5.

By pivoting the first arm segment 7 with respect to the second arm segment 8 in a scissor-like manner and by pivoting the second arm segment 8 with respect to the third arm segment 10 in a scissor-like manner, the effective length of the holding arm assembly can be prolonged and shortened.

The first and second arm segments 7, 8 can pivot with respect to each other in a free manner, i.e. more than 360°. They can be brought to a true 0° relative rotational position. The same applies for the second arm segment 8 with respect to the third arm segment 10.

The first joint axis 12 of the first arm segment joint 9 and the second joint axis 13 of the second arm segment joint 11 are directed to a region 15 in which the patient is to be treated, i.e. at least a part of the patient is to be positioned for treatment. Also the horizontal axis is directed to said region. The first joint axis 12, the second joint axis 13 and the horizontal axis 6 basically remain directed to said region 15 when the arm segments 7, 8, 10 are pivoted. Accordingly, the third arm segment 10 basically maintains its distance to said region when it is pivoted around the horizontal axis. Also the second arm segment 8 and the first arm segment 7 basically maintain their distance to said region 15 when they are pivoted around their respective joint axes 13, 12.

The first joint axis 12 is inclined with respect to the second joint axis 13 so that the first joint axis 12 is moved along a cone shell when the first arm segment joint 9 is pivoted around the second joint axis 13. The second joint axis 13 is inclined with respect to the horizontal axis 6 so that the second joint axis 13 of the second arm segment joint 11 is moved along a cone shell when the second arm segment joint 11 is pivoted around the horizontal axis 6. In this embodiment, the first joint axis 12 and the second joint axis 13 are even intersecting, and the horizontal axis 6 and the second joint axis 13 are intersecting.

The second arm segment 8 is shorter than the third arm segment 10. The second and third arm segments 8, 10 have corresponding C shapes. Also the first arm segment 7 has a C shape. When the second and third arm segments 8, 10 are in a folded up relative position, a distance between them is smaller than a distance between first and second arm segment 7, 8 when first and second arm segments 7, 8 are in a folded up relative position.

The scissor-like relative movement of the first, second and third arm segment 7, 8, 10 is carried out generally along a circumferential direction of a hypothetical sphere layer surrounding said region 15 in which the patient is to be treated. This means that the therapy head moves and remains in a kind of orbit when the arm segments 7, 8, 10 are pivoted with respect to each other, i.e. moves across to said region 15. The therapy head is guided for a kind of encompassing movement.

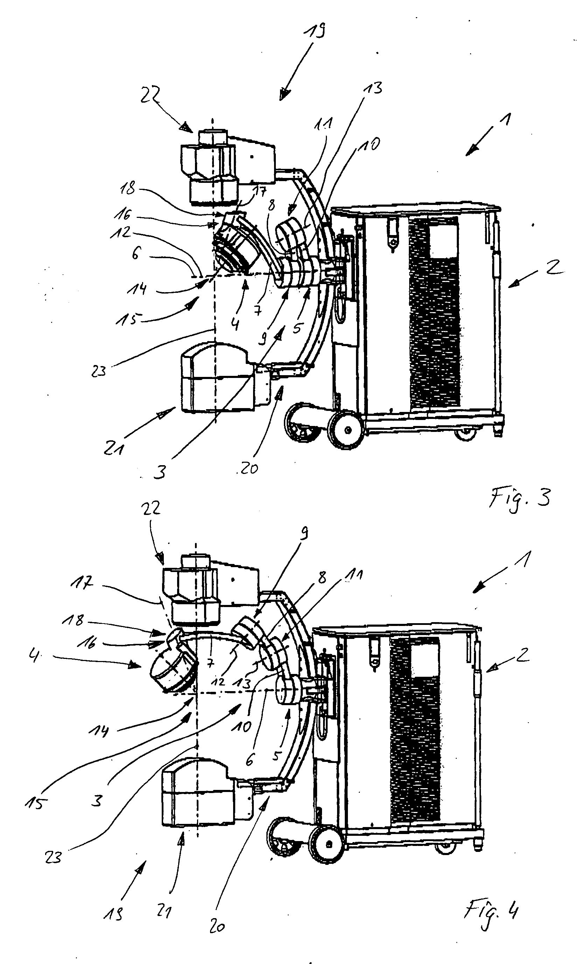

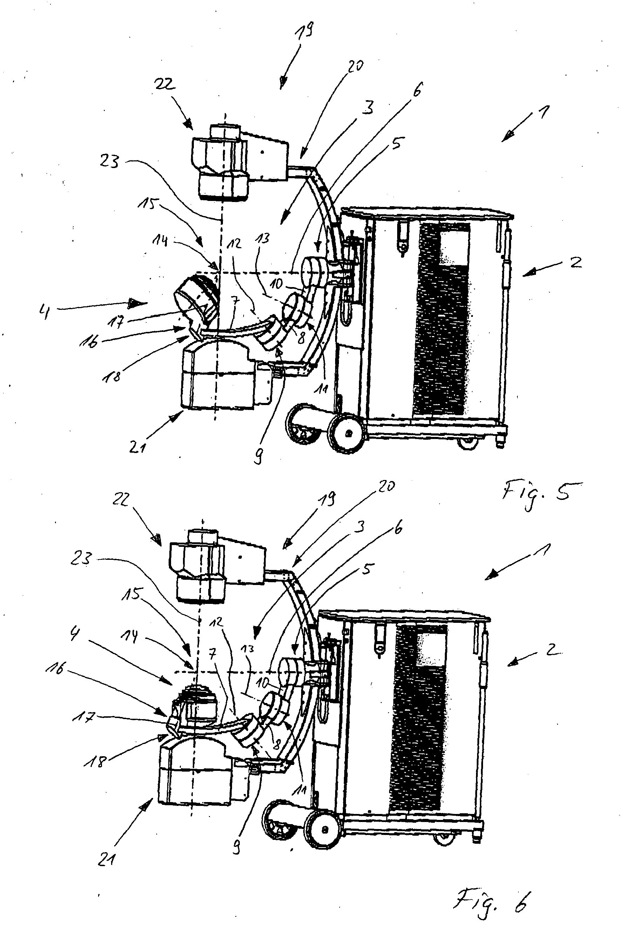

In Figs 1 and 3, the arm segments 7, 8 and 10 are in folded up relative positions while the therapy head 4 is in a first lateral treatment position in which it faces the base unit 2 with respect to a patient or said region 15. By pivoting the arm segments into stretched relative positions the therapy head 4 can be brought into a second lateral treatment position in which it opposes the base unit 2 with respect to the patient or said region 15 as becomes clear from Figs 4 and 5.

It might be necessary to make a relative movement between the patient and the base unit 2 when a first lateral side of the patient is to be reached via the first lateral treatment position of the therapy head 4 and a second lateral side of the patient is to be reached via the second lateral treatment position of the therapy head 4. This need may depend on the manner in which the patient lays on a patient table. The relative movement can be made, for instance, by moving the whole lithotripter 1 towards and away from the patient table, i.e. towards and away from the patient, or vice versa.

It is also possible to have a holding arm assembly in which the focus of the therapy head moves relative to the base unit when the therapy head is moved between a first and second lateral treatment position. If the focus changes its distance to the base unit, it might be possible to reach different lateral sides of the patient with the therapy head while maintaining the patient's relative position to the base unit unchanged.

The therapy head 4 is mounted to the first arm segment 7 via a slant joint 16 so that the therapy head is pivotable with respect to the first arm segment 7 about a slant axis 17 between a position in which it is retracted from an end 18 of the first arm segment 7 as shown in Figs 1 and 3, and a position in which it extends beyond said end 18 as shown in Fig. 2. In the present embodiment, the therapy 4 is to be pivoted from said retracted position to said extending position if it is to be brought from a first lateral treatment position as shown in Figs 1 and 3 to a second lateral treatment position as shown in Figs 4 and 5. However, it is also possible to adapt the holding arm assembly in a manner in which the therapy head 4 can be moved between both lateral treatment positions only by pivoting the arm segments with respect to each other. For instance, the first arm segment and the second arm segment could be made longer, or the second arm segment and the third arm segment could be made longer.

As becomes clear from Figs 1 through 3 in which the three arm segments are shown in folded up relative positions, the three arm segments 7, 8, 10 are arranged in a consecutive manner in the direction towards said region 15. One could alternatively say that the mid points of at least two of the first arm segment joints 9, the second arm segment joint 11 and the horizontal joint 5 are provided in sections of hypothetical shells arranged in a consecutive manner in a direction towards said region 15. It might be that one shell at least partially accommodates an adjacent shell. In the present embodiment, each mid point of the three joints 8, 11, 5 is in its own consecutive shell section.

The first joint axis 12, the second joint axis 13 and the horizontal axis 6 can be brought into at least one relative arrangement in which they are positioned in one plane. They are positioned in one plane when the three arm segments are in complete folded up relative positions, i.e. can be in one plane when the therapy head in a first lateral treatment position, and they can be in one plane when the three arm segments are in completely stretched relative positions, i.e. when the therapy head is in a second lateral treatment position.

In the present case, a plane in which the three axes 12, 13 and 6 positioned when said three arm segments 7, 8 and 10 are in completely folded up relative positions can be the same as a plane in which said three axes 12, 13 and 6 can be positioned when the three arm segments 7, 8 and 10 are in completely stretched relative positions. That is, there are at least two different relative positions of first joint axis 12, second joint axis 13 and horizontal axis 6 in which these axes are positioned within one and the same plane.

When the three arm segments 7, 8 and 10 are in a completely folded up relative position so that the two joint axes 12, 13 and the horizontal axis 6 are in one plane, the second joint axis 13 can be moved out of that plane while the first joint axis 12 and the horizontal axis 6 remain in that plane. In other words, the first joint axis 12 and the horizontal axis 6 are coinciding when the second arm segment 8 and the third arm segment 10 are in a completely folded up relative position so that the second arm segment 8 and the third arm segment 10 can be pivoted as a unit relative to the first arm segment 7 while the first arm segment 7 is not moved hereby. For example, the second arm segment join 11 can be pivoted from a position below the horizontal axis 6 or the first joint axis 12 as shown in Fig. 1 to a position above the horizontal axis 6 or the first joint axis 12 without moving the first arm segment joint 9 hereby.

The holding arm assembly has an isocentrical kinematic by means of which the therapy head 4 can be moved isocentrically. A focus 14 of the therapy head and the isocentre coincide. As the two joint axes 12, 13 are passing the isocentre, the therapy head can be isocentrically moved between first and second treatment positions. Also the horizontal axis 6 passes through the isocentre so that the therapy head can be isocentrically moved between over-table and under-table positions. Also the slant access 17 passes through the isocentre which makes it possible to isocentrically pivot the therapy head 4 between its retracted position as, for instance, shown in Fig. 1 and its extending position as, for instance, shown in Fig. 2. The focus 14 can be positioned within said region 15 in which the patient is to be treated.

The lithotripter 1 is equipped with an X-ray device 19 comprising a C-arm 20 as well as an X-ray source 21 and image device 22 provided opposing each other on ends of said C-arm 20. A central X-ray beam 23 passes through said isocentre so that therapy head 4 and X-ray device 19 are properly aligned with respect to each other even if the therapy head 4 is isocentrically moved.

The lithotripter can alternatively be provided without said X-ray device 19.

In an alternative embodiment, the holding arm assembly may comprise only two arm segments. For instance, the third arm segment of the above-described embodiment could be omitted with the second arm segment being fixed to a horizontal joint. The first arm segment and the second arm segment could be made longer than in the above embodiment. Such a holding arm assembly is also capable of moving the therapy head between over-table and under-table positions as well as between the first and second lateral treatment positions. Therein, a slant joint does not necessarily need to be provided.

The horizontal joint can be omitted in the above-described embodiments. It is, nevertheless, possible to realise the holding arm assembly in a manner in which the therapy head can at least be moved between first and second lateral treatment positions.

The thought to realize a moveability of the therapy head between a first lateral treatment and a second lateral treatment position can be independent from the thought to provide a moveability of the therapy head between an over-table position and an under-table position. There can be constructions in which the therapy head can be moved between a first lateral treatment position and a second lateral treatment position while remaining in an over-table position. In an alternative, same can be applied if only an under-table position can be attained with the kinematics of the design, even if there is no capability to move the therapy head between an under-table position and an over-table position at all. There can even be constructions without a horizontal joint.

## Claims

1. Lithotripter (1) with a base unit (2) and C-shaped holding arm assembly (3) supporting a therapy head (4), wherein a horizontal joint (5) is provided by means of which the therapy head (4) is pivotable between an over-table position and an under-table position, the holding arm assembly comprising arm segments (7, 8, 10) pivotably joined with each other,
**characterised in that** said arm segments are pivotably joined with each other in a way that the therapy head (4) is movable between a first lateral treatment position facing the base unit (2) and a second lateral treatment position opposing the base unit (2) with respect to a patient.

2. Lithotripter according to claim 1, **characterised in that the** holding arm assembly (3) comprises at least two arm segments (7, 8, 10) which are pivotable in a scissor-like manner.

3. Lithotripter according to claim 1 or 2, **characterised in that** a scissor-like relative movement of at least two arm segments (7, 8, 10) of the holding arm assembly (3) can be carried out along a hypothetical sphere layer surrounding a region (15) in which the patient is to be treated.

4. Lithotripter according to at least one of the preceding claims, **characterised in that** the axis (12, 13) of at least one joint (9, 11) pivotably connecting two arm segments (8, 9; 9, 11) in a scissor-like manner is generally directed to a region (15) in which the patient is to be treated.

5. Lithotripter according to at least one of the preceding claims, **characterised in that** at least two arm segments (7, 8, 10) which are pivotable in a scissor-like manner are positioned consecutively in a direction towards a region (15) in which the patient is to be treated, at least when these arm segments are in a folded up relative position.

6. Lithotripter according to at least one of the preceding claims 5, **characterised in that** the axis (12, 13) of at least one joint (9, 11) pivotably connecting two arm segments (7, 8; 8, 10) in a scissor-like manner is movable along a cone shell.

7. Lithotripter according to at least one of the preceding claims, **characterised in that** a pair of two arm segments (9, 10) which are pivotable in a scissor-like manner is pivotably provided between the base unit (2) and an arm segment (7) which support the therapy head (4).

8. Lithotripter according to claim (7), **characterised in that** the axes (12, 13) of the joints (9, 11) connecting said three arm segments (7, 8; 8, 10) are generally directed to a region (15) in which the patient is to be treated.

9. Lithotripter according to claim 7 or 8, **characterised in that** the axes (12, 13) of the joints (9, 11) connecting said three arm segments (7, 8; 8, 10) and the horizontal axis (6) can be moved into one plane.

10. Lithotripter according to claim 9, **characterised in that** said axes (12, 13, 6) can be in one plane when the therapy head (4) is in the first lateral treatment position and/or when the therapy head (4) is in the second lateral treatment position.

11. Lithotripter according to claim 10, **characterised in that** the plane in which said axes (12, 13, 6) can be when the therapy head (4) is in the first lateral treatment position and the plane in which said axes (12, 13, 6) can be when the therapy head (4) is in the second lateral treatment position are identical.

12. Lithotripter according to at least one of claims 9 through 11, **characterised in that** the axis (13) of that joint (11) connecting the arm segments constituting said pair of two arm segments (8, 10) can be moved out of said plane while the other axes (12, 6) remain in said plane, preferably when the therapy head is in the first lateral treatment position.

13. Lithotripter according to at least one of claims 7 through 12, **characterised in that** the joints (9, 11) linking said three arm segments (7, 8, 10) are provided in sections of hypothetical consecutive shells, wherein one shell section accommodates the other shell section at least partially.

14. Lithotripter according to at least one of the preceding claims, **characterised in that** the horizontal joint (6) and at least one joint (13) linking arm segments (8, 10) are provided in sections of hypothetical consecutive shells, wherein one shell section accommodates the other shell section at least partially.

15. Lithotripter according to at least one of the preceding claims, **characterised in that** the holding arm assembly (3) comprises an isocentrical kinematic by means of which the therapy head is isocentrically movable between the first and second treatment positions.

16. Lithotripter according to at least one of the preceding claims, **characterised in that** the horizontal axis (6) and all axes of the joints (9, 11) linking arm segments (7, 8, 10) of the holding arm assembly (3) are passing through a focus (14) of the therapy head (4).
